# FASCICULE DE BREVET EUROPEEN

(11) **EP 1 634 072 B1**
(45) Date de publication et mention de la délivrance du brevet: **27.06.2007**
(21) Numéro de dépôt: 04767333.0
(22) Date de dépôt: 11.06.2004
(51) Int. Cl.: G01N 33/50

(54) **MODELES DE SYSTEME OSSEUX**
MODELLE FÜR EIN SKELETTSYSTEM
MODELS FOR A SKELETAL SYSTEM

(30) Priorité: 11.06.2003 FR 0307025; 23.03.2004 FR 0402993
(43) Date de publication de la demande: 15.03.2006
(73) Titulaire: CENTRE NATIONAL DE LA RECHERCHE SCIENTIFIQUE (CNRS), 75794 Paris Cedex 16 (FR); ECOLE NORMALE SUPERIEURE DE LYON, 69364 Lyon Cedex 07 (FR)
(72) Inventeur: JURDIC, Pierre, F-69003 Lyon (FR); DESTAING, Olivier, 06511 New Haven, CT (US); SALTEL, Frédéric, 01210 Ferney Voltaire (FR); BONNELYE, Edith, Anne-Marie, F-69008 Lyon (FR)
(74) Mandataire: Breese Derambure Majerowicz
(86) Numéro de dépôt international: PCT/FR2004/001470
(87) Numéro de publication internationale: WO 2004/111643

(56) Documents cités:
- WO-A-02/072842
- US-A- 4 485 097
- SUN J-S ET AL: "The influence of hydroxyapatite particles on osteoclast cell activities" JOURNAL OF BIOMEDICAL MATERIALS RESEARCH 15 JUN 1999 UNITED STATES, vol. 45, no. 4, 15 juin 1999 (1999-06-15), pages 311-321, XP002305029 ISSN: 0021-9304
- LANGSTAFF S ET AL: "Resorbable bioceramics based on stabilized calcium phosphates. Part II: evaluation of biological response" BIOMATERIALS, ELSEVIER SCIENCE PUBLISHERS BV., BARKING, GB, vol. 22, no. 2, 15 janvier 2001 (2001-01-15), pages 135-150, XP004236387 ISSN: 0142-9612
- ROVIRA A ET AL: "Colonization of a calcium phosphate/elastin-solubilized peptide-collagen composite material by human osteoblasts" BIOMATERIALS, ELSEVIER SCIENCE PUBLISHERS BV., BARKING, GB, vol. 17, no. 15, 1996, pages 1535-1540, XP004032703 ISSN: 0142-9612
- DOGLIOLI, PIERRE ET AL: "A novel spectrofluorometric technique for specific biocompatibility testing of implantable materials by cell culture: report on use for multiparameter analysis of human osteoblasts cultured on commercially pure titanium and hydroxyapatite" CYTOTECHNOLOGY , 35(2), 93-100 CODEN: CYTOER; ISSN: 0920-9069, 2001, XP008032277
- MIZUNO, MORIMICHI ET AL: "Cross-linked collagen gel spheres as a useful carrier for cell culture of MC 3 T 3-E 1 clonal osteogenic cells" SHIKA KISO IGAKKAI ZASSHI , 30(6), 855-8 CODEN: SHKKAN; ISSN: 0385-0137, 1988, XP008032278
- YAMANOUCHI K ET AL: "BONE FORMATION BY TRANSPLANTED HUMAN OSTEOBLASTS CULTURED WITHIN COLLAGEN SPONGE WITH DEXAMETHASONE IN VITRO" JOURNAL OF BONE AND MINERAL RESEARCH, NEW YORK, NY, US, vol. 5, no. 16, mai 2001 (2001-05), pages 857-867, XP001064413 ISSN: 0884-0431
- MULARI M T K ET AL: "Osteoblast-like cells complete osteoclastic bone resorption and form new mineralized bone matrix in vitro" CALCIFIED TISSUE INTERNATIONAL 2004 UNITED STATES, vol. 75, no. 3, 2004, pages 253-261, XP002305030 ISSN: 0171-967X
- SHIBUTANI TOSHIAKI ET AL: "Use of glass slides coated with apatite-collagen complexes for measurement of osteoclastic resorption activity" J BIOMED MATER RES; JOURNAL OF BIOMEDICAL MATERIALS RESEARCH 2000 JOHN WILEY & SONS INC, NEW YORK, NY, USA, vol. 50, no. 2, 2000, pages 153-159, XP002305031
- SUN JUI-SHENG ET AL: "Influence of hydroxyapatite particle size on bone cell activities: An in vitro study" JOURNAL OF BIOMEDICAL MATERIALS RESEARCH, vol. 39, no. 3, 5 mai 1998 (1998-05-05), pages 390-397, XP002305032 ISSN: 0021-9304
- KIKUCHI M ET AL: "Self-organization mechanism in a bone-like hydroxyapatite/collagen nanocomposite synthesized in vitro and its biological reaction in vivo" BIOMATERIALS, ELSEVIER SCIENCE PUBLISHERS BV., BARKING, GB, vol. 22, no. 13, juillet 2001 (2001-07), pages 1705-1711, XP004245928 ISSN: 0142-9612
- JONES S J ET AL: "Simulation of bone resorption-repair coupling in vitro" ANATOMY AND EMBRYOLOGY, vol. 190, no. 4, 1994, pages 339-349, XP008038542 ISSN: 0340-2061
- TRAIANEDES KATHY ET AL: "5-Lipoxygenase metabolites inhibit bone formation in vitro" ENDOCRINOLOGY, vol. 139, no. 7, juillet 1998 (1998-07), pages 3178-3184, XP002305033 ISSN: 0013-7227

## Description

La présente invention se rapporte à des modèles de système osseux.

Elle vise plus particulièrement des modèles de système osseux in vitro comportant une matrice résorbable, des ostéoclastes et des ostéoblastes, une méthode de sélection des matrices utilisables pour les modèles selon l'invention ainsi que des modèles de système osseux mimant une pathologie particulière.

L'os est constitué de cellules et d'une matrice extracellulaire qui est minéralisée. La population cellulaire est composée de deux types cellulaires : les ostéoclastes qui dégradent la matrice osseuse et les ostéoblastes qui la reconstruisent. Jusqu'à présent, la majorité des travaux de recherche effectuée sur le sujet s'est orientée sur l'étude spécifique des ostéoclastes, en tant que cellules osseuses responsables de la dégradation de la matrice osseuse, sur l'étude spécifique des ostéoblastes ou, sur le choix de matrices artificielles capables de mimer la matrice osseuse humaine. En particulier, les articles par Shibutani et al (J Biomed Mater Res, Use of glass slides coated with apatite-collagen complexes for measurement of osteoclastic resorption activity, 31:43-49, 1996), Langstaff et al (Biomaterials, Resorbable bioceramics based on stabilized calcium phosphates, 22, 2: 135-150, 2001) et Rovira et al (Biomaterials, Colonization of a calcium phosphate/elastin-solubilized peptide-collagen composite material by human osteoblasts, 17, 15,: 1535-1540, 1996) décrivent des exemples de matrice minéralisée à base de collagène.

Plus récemment, un article par Sun et al (J Biomed Mater Res, The influence of hydroxyapatite particles on osteoclast cell activities, 45(4) :311-21, 1999) étudie l'influence de la taille des particules de poudre d'hydroxyapatite sur l'activité des ostéoclastes. Une co-culture de cellules ostéoblastiques et ostéoclastiques y est en particulier décrite.

Les travaux des inventeurs les ont amené à mettre au point un modèle de système osseux comportant une matrice minéralisée et des ostéoclastes, caractérisé en ce que des ostéoblastes sont déposés sur la matrice de manière à former un tapis à confluence et/ou des nodules, les ostéoclastes étant déposés sur ledit tapis et/ou lesdits nodules.

La disposition des deux types cellulaires est particulièrement importante pour reconstituer ce système osseux servant de modèle. En effet, les cellules osseuses humaines ne sont activées que dans un certain environnement. Les inventeurs ont réussi à reconstituer cet environnement en réalisant un tapis d'ostéoblastes à confluence ou des nodules d'ostéoblastes, et à poser sur ce tapis ou ces nodules, des ostéoclastes. De manière inattendue, les inventeurs ont constaté que les ostéoclastes, cellules 10 fois plus grandes environ que les ostéoblastes, étaient capables de se frayer un chemin à travers la population jointive d'ostéoblastes (sous forme de tapis à confluence ou de nodules) afin d'aller exercer leur activité de résorption directement sur la matrice osseuse. Les ostéoclastes se trouvent alors localisés sous la population d'ostéoblastes, et ce, au bout de la 2^{e} heure environ, qui suit le dépôt des ostéoclastes.

Afin de vérifier que la migration des ostéoclastes à travers la couche ostéoblastique est bien un mécanisme spécifique du tissu osseux, les inventeurs ont repris l'expérience à l'identique sur une lamelle de dentine (support le plus proche de l'os). Or, sur ce support, la migration à travers la couche ostéoblastique a également été observée.

Une telle constatation permet donc de valider le modèle comme adéquat pour mimer le système osseux, et plus particulièrement, le système osseux murin ou humain. D'autres observations, décrites dans les exemples de la présente demande, confirment cette validation.

Avantageusement, le modèle de système osseux selon l'invention comporte une matrice composée de collagène et de phosphate de calcium et/ou des dérivés de phosphate de calcium. De préférence, le dérivé de phosphate de calcium est de l'hydroxyapatite.

De préférence, le rapport entre les ostéoclastes et les ostéoblastes est d'environ 1/10 à 1/25. Ce rapport est variable car dépendant du phénomène que l'on souhaite observer. En effet, si un trop grand nombre d'ostéoclastes est ajouté, il se produira une dégradation trop importante et trop rapide de la matrice, gênant ainsi une éventuelle quantification du matériel résorbé (mesure de la surface qui n'est plus minéralisée).

Le modèle selon l'invention fournit les moyens de tester l'efficacité de drogues connues ou nouvelles dans la perspective de la mise en évidence de nouveaux traitements thérapeutiques dans un contexte.osseux normal ou pathologique.

Par « drogues », on entend des molécules biologiquement actives.

Plus particulièrement, l'invention permet de tester le potentiel de toutes drogues déjà connues (ex : biphosphonate, PTH, vitamine D...) ou nouvelles sur la formation osseuse (ostéoblastes) et /ou sur l'invasion et /ou sur la migration et /ou sur la résorption osseuse (ostéoclastes) générant ainsi un test rapide in vitro d'évaluation du potentiel thérapeutique de toutes molécules pouvant agir sur le métabolisme osseux, mais aussi des effets néfastes (effet secondaires) de toutes drogues utilisées pour d'autres pathologies ne touchant pas l'os (ex : diabètes, maladies cardiaques...).

Dans la demande, le terme « migration » employé seul signifie le mouvement des ostéoclastes durant la résorption. De même, le terme « invasion » employé seul renvoie à la colonisation du support à résorber. En revanche, lorsque ces termes sont utilisés pour faire référence à la traversée de la couche ostéoblastique, ils sont suivis d'une expression le précisant.

Selon un mode de réalisation, les ostéoblastes et/ou des ostéoclastes déposés peuvent être génétiquement modifiés. Le dépôt de cellules génétiquement modifiées permet l'étude du comportement de ces cellules et de l'évolution du système osseux, plus particulièrement en vue d'une thérapie génique. L'utilisation de ces cellules génétiquement modifiées est particulièrement adéquate sur les modèles de systèmes osseux présentant une pathologie, tels que décrits par la suite.

La présente invention vise également une méthode de sélection de matrices permettant de reconstituer un modèle de système osseux,
caractérisée en ce que l'on soumet une matrice minéralisée au procédé suivant :
- dépôt d'un tapis et/ou des nodules d'ostéoblastes à confluence sur la matrice,
- dépôt d'ostéoclastes isolés sur le tapis et/ou les nodules,
- observation de l'invasion des ostéoclastes à travers le tapis et/ou les nodules d'ostéoblastes,
- observation de la résorption de la matrice minéralisée,
- sélection des matrices sur lesquelles les ostéoclastes sont localisés entre la matrice et le tapis et/ou les nodules d'ostéoblastes et sur lesquelles une résorption est observée ;
ainsi que les matrices artificielles sélectionnées à l'aide de ladite méthode.

En effet, le comportement des cellules sur la matrice permet de déterminer si l'environnement choisi pour mimer le système osseux est adéquat. Par l'observation du comportement cellulaire, on peut donc déterminer si la matrice choisie est un bon modèle de matrice osseuse.

Avantageusement, le matériau de la matrice à tester pourra être choisi parmi l'ensemble des biomatériaux, c'est-à-dire des matériaux compatibles avec le tissu vivant. Une modification de la matrice (ajout de différents composés protéiques ou autres) peut amener au développement de nouveaux biomatériaux.

Comme nous l'avons évoqué plus haut, l'invention propose également des modèles de système osseux mimant des pathologies osseuses. Ces modèles sont réalisés de préférence à partir de cellules (ostéoblastes et/ou ostéoclastes) extraites de tissus provenant de toutes pathologies osseuses.

En particulier, l'invention propose un modèle de système osseux cancéreux, atteint d'ostéoporose, d'ostéomalacie et/ou d'arthrite rhumatoïde.

Par « modèle de système osseux cancéreux », on entend les modèles de système osseux correspondant aux pathologies suivantes :
- un malade atteint d'une tumeur primaire cancéreuse,
- un malade atteint d'une tumeur primaire cancéreuse (sein, prostate, ...) avec métastases,
- un malade atteint d'un cancer des os.

Ces modèles mimant des pathologies utilisent le modèle de système osseux décrit plus haut, mais portant un certain nombre de modifications.

Par exemple, pour le modèle de système osseux cancéreux, les modifications sont les suivantes :
- les ostéoblastes et/ou les ostéoclastes sont issus d'animaux normaux, ovariectomisés et/ou orchiectomisés, etc..., et,
- on dépose de plus des cellules issues de lignées cellulaires de cancer.

Pour étudier un cancer des os, les cellules déposées seront par exemple issues d'une lignée cellulaire de cancer des os. Dans les autres cas, on déposera des cellules issues d'une lignée cellulaire de tumeur primaire à potentiel métastatique (sein, prostate, ...) ou non.

Ce modèle permet d'observer les phénomènes de colonisation du tissu osseux par les cellules tumorales et de visualiser la phase de métastase. En effet, comme le montre l'invasion des ostéoclastes à travers la couche ostéoblastique, les cellules placées dans un environnement mimétique du système osseux, ont la capacité de se déplacer dans cet environnement.

Ce modèle est plus particulièrement adapté pour réaliser un test d'agressivité des cellules tumorales (voir exemple 4). L'existence de nombreux cancers primaires (sein , prostate ....) capables de métastaser dans l'os est maintenant un fait établi en cancérologie. Or, ce cancer de l'os qui en découle s'avère dans la majorité des cas, incurable. L'invention permet de tester in vitro l'agressivité (invasion, migration, prolifération) de cellules' tumorales (par exemple, issues de tumeurs mammaires ou de la prostate) provenant de biopsies de patients. Ces cellules tumorales sont déposées sur le modèle de système osseux selon l'invention et permettent ainsi d'estimer le potentiel agressif des cellules de la tumeur primaire au niveau de leur capacité d'invasion, de migration et/ou de prolifération ainsi que d'établir un pronostic sur le développement d'un cancer secondaire osseux.

Plus précisément, l'invention propose un test rapide in vitro d'évaluation du potentiel thérapeutique (chimiothérapie et/ou radiothérapie) de toutes molécules pouvant être utilisées en cancérologie afin de réduire l'apparition et/ou de contribuer au traitement anti-cancéreux d'un cancer de l'os.

Le modèle de système osseux atteint d'ostéoporose comporte les modifications suivantes :
- les ostéoblastes et/ou les ostéoclastes sont issus d'animaux normaux, ovariectomisés et/ou orchiectomisés, l'ostéoporose étant alors induite chimiquement *in situ* et/ou d'animaux knock-out, transgéniques pour toutes molécules dont la modulation de l'expression induit une baisse de la masse osseuse,
tandis que le modèle de système osseux atteint d'ostéomalacie est modifié comme suit :
- les ostéoblastes et/ou les ostéoclastes sont issus d'animaux normaux, l'ostéomalacie étant alors induite chimiquement *in situ* et/ou knock-out pour le récepteur à la vitamine D ou pour toutes autres molécules capable d'induire une ostéomalacie.

De nombreuses molécules sont connues pour induire une ostéoporose. On peut citer, à titre d'exemple, la dexaméthasone, l'hydrocortisone, la prednisolone et ses dérivés, la fluocortolone, l'héparine calcique ou sodique.

De même, l'ostéomalacie peut être induite par les molécules suivantes : les sels d'aluminium, le barbital et ses dérivés, le rétinol, le bêtacarotène.

Enfin, le modèle de système osseux atteint d'arthrite rhumatoïde peut comporter les modifications suivantes
- les ostéoblastes et/ou les ostéoclastes sont issus d'animaux normaux, l'arthrite rhumatoïde étant alors induite chimiquement *in situ,* et/ou d'animaux knock-out, transgéniques pour toutes molécules capables d'induire une arthrite rhumatoïde ou d'animaux ayant subi des injections de collagène de type II, ou de toutes autres substances susceptibles d'induire une inflammation articulaire mimant une arthrite rhumatoïde.

Les molécules pouvant induire des arthrites rhumatoïdes sont, à titre d'exemple, certains interférons α, certains vaccins (BCG, hépatite B, rubéolique...), le cortivazol, certains sels de lithium, l'ampicilline.

La présente demande protège également l'utilisation des différents modèles pour réaliser des criblages de molécules thérapeutiques et des tests d'efficacité. A l'aide des modèles selon l'invention, l'efficacité des différentes molécules actuellement connues et de celles qui seront mises à jour pourra être comparée. A titre d'exemple, le test d'efficacité pour l'ostéoporose pourra mettre en comparaison des molécules connues pour rétablir la masse osseuse comme le biphosphonate, les oestrogènes et toutes les nouvelles molécules mises à jour à l'aide du modèle utilisé pour réaliser un criblage. Le test d'efficacité pour l'ostéomalacie pourra prendre comme molécule de référence la vitamine D, tandis que le test pour l'arthrite rhumatoïde pourra prendre comme molécules de référence l'aspirine et/ou les anti-inflammatoires non stéroïdien.

De manière avantageuse, les modèles selon l'invention sont particulièrement adaptés pour réaliser des tests de toxicité d'un composé chimique dans lesquels au moins une concentration dudit composé est testée sur un modèle selon l'invention. De tels tests permettent d'évaluer les effets secondaires de médicaments sur la physiologie osseuse (par exemple, les médicaments contre le diabète, les antibiotiques), les effets toxiques des polluants (dioxine, insecticides...), etc... Avantageusement, plusieurs concentrations seront testées afin d'établir une relation entre la concentration du composé et les effets secondaires engendrés dans le modèle de système osseux.

Un modèle de système osseux atteint d'ostéomyélite et/ou d'infection osseuse peut également être construit en ajoutant dans le système modèle selon l'invention différentes souches bactériennes ou virales. A titre d'exemple, on citera les souches suivantes : *Enterobacter cloacae,* le staphylocoque doré, le streptocoque bêta hémolytique A, *Haemophilu s influenzae type b,* les salmonelles, *Pseudomonas,* et/ou les pneumocoques...

D'autres caractéristiques et avantages de l'invention apparaîtront dans les exemples qui suivent, avec références aux figures, qui représentent respectivement :

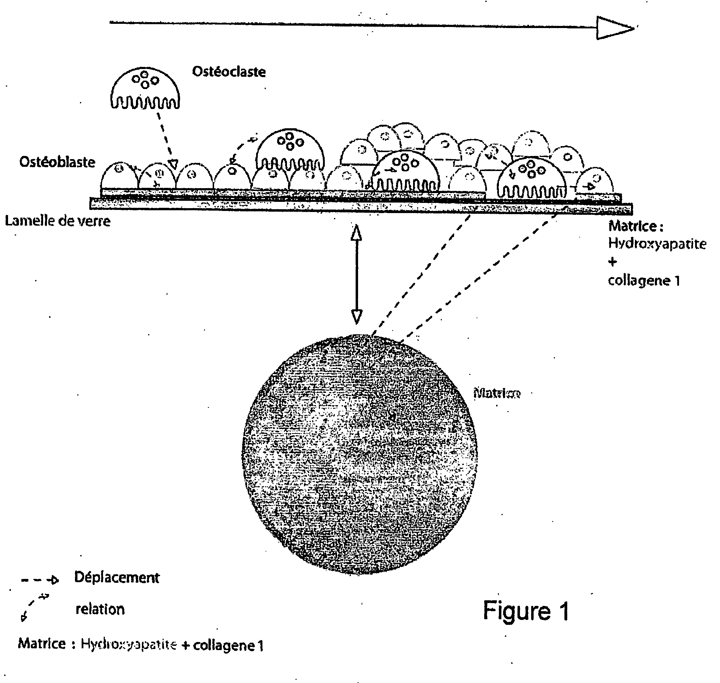

- la figure 1, un schéma du processus de traversée de la couche ostéoblastique par les ostéoclastes et de résorption ostéoclastique,
- les figures 2 et 3, des images obtenues par analyse 3D par microscopie confocale montrant l'agencement des deux types cellulaires, A : ostéoclaste, B : ostéoblaste, C : coupe en Z, et,
- la figure 4, des images obtenues par analyse 3D par microscopie confocale montrant l'agencement des deux types cellulaires selon la coupe en Z, afin de déterminer les effets du PP2 (fig. 4B) en comparaison avec l'essai témoin (fig. 4A).

### Exemple 1 : Reconstitution d'une interface osseuse cellularisée pour un test de résorption - Tapis d'ostéoblastes à confluence.

### a. Matrice

La matrice minéralisée est préparée soit sur lamelles de verre (pour microscopie) soit sur plastique traité pour culture cellulaire. Le support est d'abord recouvert pendant 15h d'une solution de collagène I à 0,1mg/mL diluée dans de l'acide acétique 0,1M. L'excès de collagène est éliminé puis le support est recouvert pendant une semaine à 37°C avec une solution de TRIS 200mM, pH(8,5), 0,4g/L de phosphatase alcaline, 0,4g/L de phosvitine et 3g/L de chlorure de diméthyle suborimidate.

Cette étape est suivie de la minéralisation proprement dite. Cette minéralisation est constituée de 2 étapes successives :
1- le support est recouvert d'une solution TRIS 200mM, pH(8,5), 0,4g/L de phosphatase alcaline, 0,4g/L de phosvitine pendant 3h, puis,
2- cette solution est remplacée par une solution de TRIS 200mM, pH(8,5) et de β-glycérophosphate 126,06g/L pendant 20h.

Ces deux étapes sont répétées 10 fois. La trame de collagène I peut être complétée avec d'autres protéines (ostéopontine, vitronectine, BSP, ostéocalcine, collagène de type I conjugué à un agent fluorescent, par exemple, la rhodamine, etc...) ou d'autres substituts (par exemple, des substituts minéraux : fluor, strontium ranélate...).

### b. Ostéoblastes

Des cellules de la lignée ostéoblastique murine MC 3T3 sont mises en culture en milieu α-MEM complété avec 10% en volume de sérum de veau foetal, de la dexaméthasone 10⁻⁸M et de l'acide ascorbique 0,028mM. Les cellules sont ensuite détachées et ensemencées à confluence sur le support minéralisé.

Les ostéoblastes peuvent également être issus de lignées ostéoblastiques de rat (Ros) ou humaine (HEPM, hFOB) et préparés selon le même protocole. Alternativement, un protocole de culture primaire d'ostéoblastes est proposé dans l'exemple 2.

### c. Ostéoclastes

Les ostéoclastes issus de cultures primaires humains ou murins ou de lignées (par exemple, la lignée RAW ou lignée humaine GCT23) sont obtenus après 7 jours de différenciation comme décrit dans Destaing et al (Mol Biol Cell, Podosomes display actin turnover and dynamic self-organization in osteoclasts expressing actin-green fluorescent protein, 14(2) :407-16, 2003). Les précurseurs d'ostéoclastes sont cultivés en présence de milieu α-MEM complété avec 10% en volume de sérum de veau foetal et de deux cytokines recombinantes : M-CSF et RANK-L (20ng/L). Les cellules sont placées à 37°C et 5% de CO₂ et le milieu est changé tous les deux jours pendant 7-8 jours. Les ostéoclastes différenciés sont détachés avec une solution d'EDTA à 0,25mM diluée dans du PBS 1X. Les ostéoclastes sont ensemencés à une densité de 10 cellules/mm².

### d. Fixation

La fixation est réalisée dans du PBS 1X additionné de formaldéhyde 3,7% pendant 10 min.

### e. Résultats

La résorption de la matrice minéralisée est observée en microscopie photonique dès la 6^{e} ou 7^{e} heure suivant le montage du modèle (temps écoulé entre le premier contact ostéoclastes-ostéoblastes et la résorption de la matrice).

Une seconde expérience a été menée afin de démontrer que les ostéoclastes sont capables de traverser le tapis dense de cellules ostéoblastiques avant de former des trous de résorption.

Par analyse 3D par microscopie confocale, il a été possible de démontrer le pouvoir invasif des ostéoclastes et de quantifier la résorption en mesurant la surface du substrat qui n'est plus minéralisée.

La fixation des cellules est réalisée une heure et quatre heures après le dépôt des ostéoclastes.

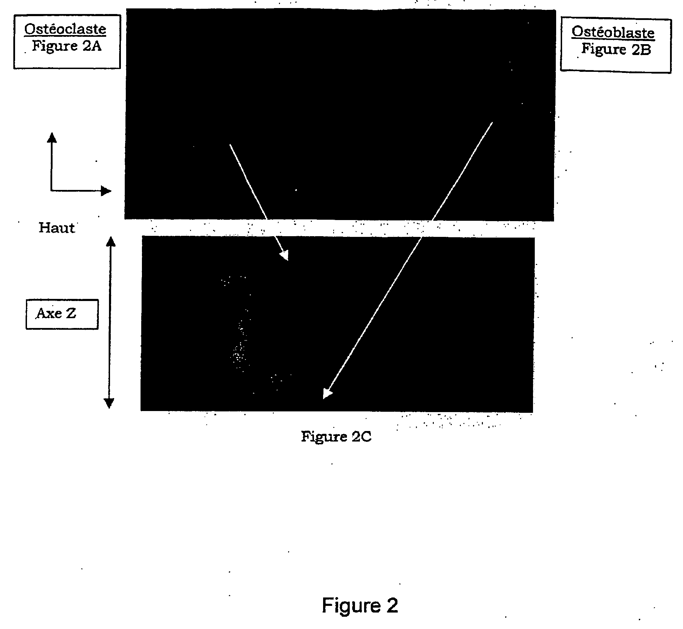

La figure 2 comporte trois images montrant la localisation des différentes cellules une heure après le dépôt des ostéoclastes.

La figure 2A. est une image de l'ostéoclaste. Celui-ci présente une forte polarité, ce qui démontre l'état actif des cellules. En effet, un o.stéoclaste déposé sur un support non adéquat présente une faible épaisseur (non polarisé). En revanche, sur un support adéquat, celui s'épaissit afin de former un pôle basal (contact avec les ostéoblastes) et un pôle apical (contact avec le milieu). Les inventeurs ont constaté que cette polarité était maintenue durant la traversée de la couche ostéoblastique et maintenue lors la résorption. Toutefois, le pôle basal se retrouve au contact de la matrice tandis que le pôle apical est au contact des ostéoblastes.

La figure 2B est une image de l'ostéoblaste. On constate la présence de nombreux filaments d'actine. Les fibres de stress d'actine constituent un marqueur présent dans les ostéoblastes MC3T3.

La figure 2C est une coupe en Z, permettant de visualiser la localisation selon l'axe Z des deux cellules photographiées précédemment. Cette image montre que l'ostéoclaste est situé au dessus du tapis ostéoblastique (ligne claire en continue).

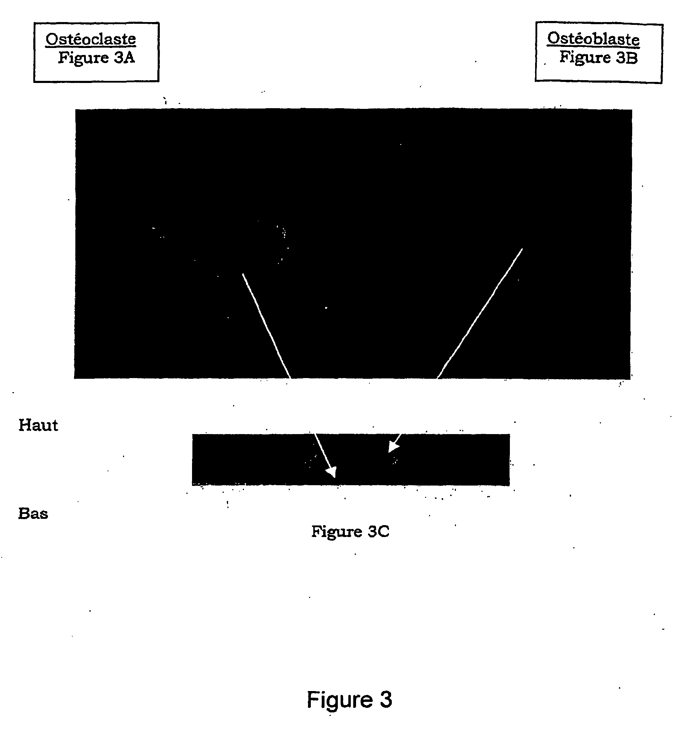

La figure 3 comporte également trois images correspondant aux trois images de la figure 2. La figure 3A est une photographie de l'ostéoclaste. Celui-ci a changé de forme, il s'est aplati. La figure 3B représente le tapis d'ostéoblastes. Celui-ci est à présent localisé au-dessus de l'ostéoclaste, ce dernier étant en contact direct avec la matrice (Figure 3C) et présente l'organisation d'un ostéoclaste en cours de résorption (la « sealing zone », structure caractéristique des cellules résorbantes).

### Exemple 2 : Reconstitution d'une interface osseuse cellularisée pour un test de résorption - Nodules d'ostéoblastes.

### a. Matrice

La matrice est préparée comme décrit dans l'exemple 1.

### b. Ostéoblastes

Les ostéoblastes utilisés peuvent être issus de cultures primaires humaines ou murines, ou de lignées comme décrit dans l'exemple 1.

Les cellules ont été isolées par digestion enzymatique (collagénase (Sigma #C-0130)) à partir de calvaria de souris de 2 jours. Les cellules obtenues des quatre dernières étapes de digestion (population II-V) sont ensuite ensemencées dans des flasques T75 dans du milieu αMEM contenant 15% de sérum de veau foetal (Flow Laboratories, McLean, VA) et 100 µg/ml pénicilline G (Sigma Chemical Co., St. Louis, MO), 50 µg/ml gentamycine (Sigma), et 0.3 µg/ml fungizone (Flow Laboratories). Après 24h d'incubation les cellules adhérentes sont rincées au PBS, traitées à la trypsine (0,01%) dans une solution saline de citrate, resuspendues dans le milieu standard décrit précédemment et ensemencées sur des plaques de 12 puits sur la matrice décrite dans l'exemple 1 à 10⁴ cellules/puit. Après 24h d'incubation, le milieu est changé et supplémenté en acide ascorbique (50 µg/ml) et en sodium β-glycérophosphate (10 mM). Le milieu est ensuite changé tous les deux jours. Toutes les plaques sont incubées à 37°C sous une atmosphère à 95% d'air et 5% de CO₂.

Une culture primaire d'ostéoblastes humains est également possible.

### c. Ostéoclastes

Les ostéoclastes sont préparés selon la méthode décrite dans l'exemple 1.

### d. Fixation

La fixation est réalisée comme dans l'exemple 1.

### e. Résultats

La résorption de la matrice minéralisée est observée en microscopie photonique dès la 7^{e} heure suivant le montage du modèle.

Une seconde expérience a été menée afin de démontrer que les ostéoclastes sont capables de traverser les nodules d'ostéoblastes avant de former les trous de résorption.

Par analyse 3D par microscopie confocale, il est possible de démontrer le pouvoir invasif des ostéoclastes et de quantifier la résorption en mesurant la surface du substrat qui n'est plus minéralisée.

Comme dans l'exemple précédent, nous avons pu observer la traversée des ostéoblastes par les ostéoclastes ainsi que les trous de résorption réalisés par les ostéoclastes.

### Exemple 3 : Variantes des types cellulaires déposés

| pathologies osseuses : | description du système |
|---|---|
| ostéoporose (dégradation de la matrice osseuse due à l'arrêt de la synthèse d'oestrogènes chez la Femme, de la testostérone chez l'Homme.) | -ostéoblastes de souris ovariéctomisées (femelle) ou orchiectomisées (mâle) + ostéoclastes normaux |
| | -ostéoblastes normaux + ostéoclastes de souris ovariectomisées (OVX) ou orchiectomisées (ORX) |
| | -ostéoblastes+ostéoclastes de souris OVX (femelle) ou ORX (mâle)/ ostéoclastes+ ostéoblastes normaux. |
| | |
| ostéomalacie (déficience en vitamine D engendrant une mauvaise minéralisation de la matrice osseuse) | -ostéoblastes de souris KO VDR (récepteur de la vitamine D) + ostéoclastes normaux |
| | -ostéoblastes normaux + ostéoclastes de souris KO VDR (récepteur de la vitamine D) |
| | -ostéoblastes+ostéoclastes de souris KO VDR (récepteur de la vitamine D)/ ostéoclastes+ ostéoblastes normaux. |
| | |
| arthrite rhumatoïde (AR) (inflammation causée par une augmentation de la résorption osseuse par les ostéoclastes) | -ostéoblastes de souris (injection collagène type II capable d'induire chez la souris in vivo une AR) ostéoclastes normaux |
| | -ostéoblastes normaux + ostéoclastes de souris (injection collagène type II capable d'induire chez la souris in vivo une AR) |
| Autres pathologies osseuses et tous KO déjà connus pour présenter un défaut dans le métabolisme osseux (ALP, src, c-fos, OPG, Rankl, récepteur des estrogène, aromatase, leptine .....) et qui pourraient être associés à des mutations humaines non encore déterminées: et tous nouveaux knock out. | -ostéoblastes+ostéoclastes de souris AR/ ostéoclastes + ostéoblastes normaux |

| | |
|---|---|
| test de l'effet de drogues connues - Oestrogènes (SERM, tamoxifène, raloxifène)- testostérone, PTH (Parathyroïde hormone), prostaglandines (PGE2), biphosphonates (alendronate), vitamines D, osteoprotegerine (OPG), RANKL, AINS (Anti-inflammatoires non stéroïdien, ex :ibuprofène), glucocorticoïdes, hormone thyroïdienne T3....... test de l'effet de nouvelles drogues | |

| pathologies cancéreuses | cellules utilisées |
|---|---|
| cancer du sein : cancer qui métastase en tumeur osseuse | -ostéoblastes et ostéoclastes normaux+ lignées cellulaires de cancers mammaires soient agressives (MDA-MB-231) soient non-agressives (MCF-7). Observation de colonisation des cellules mammaires, formation in vitro de tumeurs mammaires. |
| 1/3 des ostéoses métastatiques et plus de | |
| 2/3 des cancers féminins | |
| | |
| | -ostéoblastes et ostéoclastes anormaux (OVX)+ lignées cellulaires de cancers mammaires. Test du rôle des estrogènes dans le phénomène de cancérisation |
| | |
| cancer de la prostate: cancer qui métastase en tumeur osseuse un des plus fréquents cancers masculins | -ostéoblastes et ostéoclastes normaux+ lignées cellulaires de cancers de la prostate (LAPC-4). Observation de colonisation des cellules de la prostate, formation in vitro de tumeurs . |
| | -ostéoblastes et ostéoclastes anormaux (ORX) + lignées cellulaires de cancers de la prostate. Test du rôle de la testostérone dans le phénomène de cancérisation. |
| | |
| autres cancers pouvant métastaser en tumeurs osseuses : | -lignée cellulaire humaine d'adénocarcinome du poumon : A549 |
| | -lignées cellulaires humaine de carcinome du rein: ACHN, A704, |
| -cancer du poumon | |
| -cancer du rein | Caki-1 |
| -cancer de la thyroïde | -lignée cellulaire humaine de carcinome de la thyroide: FRO |
| -cancer digestif | -lignée cellulaire humaine de carcinome gastrique: EPG85-257P |
| -cancer de l'utérus | -lignée cellulaire humaine de carcinome de l'endomètre: HEC-1 |
| -cancer de l'ovaire | -lignée cellulaire humaine d'épithélium de cancer ovarien: EOC |

| | |
|---|---|
| test de l'effet de drogues connues pour agir sur la diminution de l'invasion et de la prolifération cellulaire : agents chimiothérapeutiques, inhibiteur de la cyclo-oxygénase (COX), mélatonine...... test de nouvelles drogues | |

### Exemple 4 : Test d'agressivité de cellules tumorales

Une femme sur 4 mourra d'un cancer du sein et un homme sur 3 d'un cancer de la prostate en France (EUCAN, cancer incidence mortality and prevalence un EU 1996).

Ces deux cancers sont généralement des cancers primaires qui lorsqu'ils ne sont pas stoppés à temps métastasent dans l'os, cancers qui s'avèrent alors irrémédiablement létaux. Les modèles de système osseux selon l'invention devraient permettre d'étudier pourquoi ces cellules mammaires ou de la prostate métastasent quasi systématiquement dans l'os.

Le système selon l'invention permet en effet de visualiser in vitro l'invasion (ou le chimiotactisme des produits sécrétés par les cellules de cette interface osseuse cellularisée) de cellules cancéreuses mammaires ou de la prostate dans la matrice osseuse. Des lignées de cellules humaines de cancers du sein agressives (MDA-MB-231) ou non-agressives (MCF-7) ou encore des lignées de cellules humaines de cancers de la prostate (LAPC-4) sont déposées sur le système selon l'invention. Cette expérience simple permet d'observer le comportement de ces cellules comparé à d'autres types de cellules connues pour peu métastaser dans l'os, d'observer les interactions entre les cellules tumorales et les cellules du systèmes osseux ou encore, la formation de masses cellulaires prolifératives, l'éventuelle dégradation de la matrice osseuse.

Une fois la formation de tumeurs in vitro réalisée, une seconde étape consiste à analyser l'efficacité de drogues anti-cancéreuses déjà connues (chimiothérapie...) ou encore à mettre en évidence par criblage de nouvelles molécules actives sur la prolifération cellulaire (diminution de la masse cellulaire ou du nombre de foci).

### Exemple 5 : Criblage de molécules, à des fins thérapeutiques

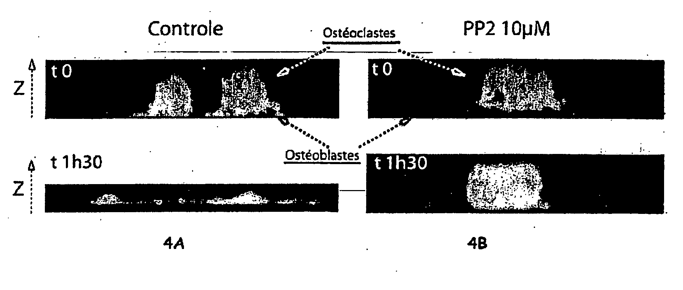

Un inhibiteur de la tyrosine kinase Src, le PP2, a été testé dans le modèle selon l'invention. Ce produit est connu pour ses propriétés anti-resorptives et semble constituer une molécule thérapeutique potentielle pour le traitement de l'ostéoporose. Pour réaliser l'interface osseuse selon l'invention, des ostéoclastes dérivés de RAW-GFP ont été déposés sur un tapis de cellules ostéoblastiques de type MC3T3. Après une durée de 1 heure à 1 heure 30min, en absence de traitement, les ostéoclastes ont traversés ce tapis cellulaire et se sont étalés sous ce dernier. En revanche, si les ostéoclastes sont traités avec l'inhibiteur de tyrosine kinase Src, PP2, à une concentration de 10 µM, les ostéoclastes ne traversent plus le tapis cellules ostéoblastiques (voir fig. 4).

Le PP2 est donc capable de bloquer les mécanismes d'invasion des ostéoclastes dans le modèle selon l'invention entraînant ainsi une diminution de la résorption osseuse (incapacité des ostéoclastes à atteindre la matrice minéralisée).

Cette expérience permet de démontrer que l'interface osseuse selon l'invention peut donc être utilisée à des fins de criblage de molécules thérapeutiques.

## Revendications

1. Modèle de système osseux comportant une matrice minéralisée et des ostéoblastes, **caractérisé en ce que** les ostéoblastes sont déposés sur la matrice de manière à former un tapis à confluence et/ou des nodules, et les ostéoclastes sont déposés sur ledit tapis et/ou lesdits nodules.

2. Modèle de système osseux selon la revendication 1, **caractérisé en ce que** la matrice minéralisée est une matrice composée de collagène et de phosphate de calcium et/ou des dérivés de phosphate de calcium.

3. Modèle de système osseux selon la revendication 1 ou 2, **caractérisé en ce que** la matrice minéralisée est une matrice composée de collagène et d'hydroxyapatite.

4. Modèle de système osseux selon l'une quelconque des revendications 1 à 3, **caractérisé en ce que** le rapport ostéoclastes sur ostéoblastes est d'environ 1/10 à 1/25.

5. Modèle de système osseux selon l'une quelconque des revendications 1 à 3, **caractérisé en ce que** les ostéoblastes et/ou des ostéoclastes déposés sont génétiquement modifiés.

6. Méthode de sélection de matrice permettant de reconstituer un modèle de système osseux, **caractérisée en ce que** l'on soumet une matrice au procédé suivant :
- dépôt d'un tapis et/ou des nodules d'ostéoblastes à confluence sur la matrice,
- dépôt d'ostéoclastes isolés sur le tapis et/ou les nodules,
- observation de l'invasion des ostéoclastes à travers le tapis et/ou les nodules d'ostéoblastes,
- sélection des matrices sur lesquelles les ostéoclastes sont localisés entre la matrice et le tapis et/ou les nodules d'ostéoblastes.

7. Méthode de sélection selon la revendication 6, **caractérisée en ce qu'**elle comporte en outre une étape d'observation de la résorption de la matrice et que l'on sélectionne les matrices sur lesquelles on observe également une résorption.

8. Modèle de système osseux selon l'une quelconque des revendications 1 à 5, **caractérisé en ce que** le modèle est, modifié comme suit :
- les ostéoblastes et/ou les ostéoclastes sont issus d'animaux normaux, ovariectomisés et/ou orchiectomisés,
- on dépose de plus des cellules issues de lignées cellulaires de cancer.

9. Modèle de système osseux selon l'une des revendications 1 à 5, **caractérisé en ce que** le modèle est modifié comme suit :
- les ostéoblastes et/ou les ostéoclastes sont issus d'animaux normaux, ovariectomisés et/ou orchiectomisés, l'ostéoporose étant alors induite chimiquement *in situ* et/ou d'animaux knock-out, transgéniques pour toutes molécules dont la modulation de l'expression induit une baisse de la masse osseuse.

10. Modèle de système osseux selon l'une des revendications 1 à 5, **caractérisé en ce que** le modèle est modifié comme suit :
- les ostéoblastes et/ou les ostéoclastes sont issus d'animaux normaux, l'ostéomalacie étant alors induite chimiquement *in situ* et/ou knock-out pour le récepteur à la vitamine D ou pour toutes autres molécules capables d'induire une ostéomalacie.

11. Modèle de système osseux selon l'une des revendications 1 à 5, **caractérisé en ce que** le modèle est modifié comme suit :
les ostéoblastes et/ou les ostéoclastes sont issus d'animaux normaux, l'arthrite rhumatoïde étant alors induite chimiquement *in situ,* et/ou d'animaux knock-out, transgéniques pour toutes molécules capables d'induire une arthrite rhumatoïde ou d'animaux ayant subi des injections de collagène de type II, ou de toutes autres substances susceptibles d'induire une inflammation:articulaire mimant une arthrite rhumatoïde.

12. Modèle de système osseux selon l'une des revendications 1 à 5 **caractérisé en ce que** le modèle est modifié comme suit :
- on ajoute dans le milieu au moins une souche bactérienne ou virale choisie parmi *Enterobacter cloacae,* le staphylocoque doré, le streptocoque bêta hémolytique A, *Haemophilus influenzae type b,* les salmonelles, *Pseudomonas*, et/ou les pneumocoques.

13. Utilisation des modèles selon les revendications 1 à 5, 8 à 12, pour tester le criblage de molécules thérapeutiques.

14. Test d'agressivité de cellules tumorales **caractérisé en ce que** des cellules tumorales d'un patient sont déposées dans un modèle selon l'une quelconque des revendications 1 à 5, 9 à 12, selon l'état pathologique du patient, afin d'observer le développement de cancer secondaire osseux.

15. Test de tonicité d'un composé chimique **caractérisé en ce que** l'on teste au moins une concentration dudit composé sur un modèle selon l'une quelconque des revendications 1 à 5, 9 à 12.

## Claims

1. A bone system model comprising a mineralized matrix and osteoblasts, **characterised in that** the osteoblasts are deposited on the matrix in order to form a confluent layer and/or nodules, and the osteoclasts are deposited on said layer and/or said nodules.

2. A bone system model according to claim 1, **characterised in that** the mineralized matrix is a matrix consisting of collagen and calcium phosphate and/or calcium phosphate derivatives.

3. A bone system model according to claim 1 or 2, **characterised in that** the mineralized matrix is a matrix consisting of collagen and hydroxyapatite.

4. A bone system model according to any one of claims 1 to 3, **characterised in that** the osteoclast to osteoblast ratio is approximately 1/10 to 1/25.

5. A bone system model according to any one of claims 1 to 3, **characterised in that** the deposited osteoblasts and/or osteoclasts are modified genetically.

6. A matrix selection method allowing a bone system model to be reconstituted, **characterised in that** a matrix is subjected to the following process:
- deposit of a confluent layer and/or nodules of osteoblasts on the matrix,
- deposit of isolated osteoclasts on the layer and/or nodules,
- observation of the invasion of osteoclasts through the layer and/or nodules of osteoblasts,
- selection of matrices on which the osteoclasts are located between the matrix and the layer and/or nodules of osteoblasts.

7. A selection method according to claim 6, **characterised in that** it further comprises a matrix resorption observation stage and that matrices are selected on which a resorption is also observed.

8. A bone system model according to any one of claims 1 to 5, **characterised in that** the model is, modified as follows:
- the osteoblasts and/or osteoclasts come from normal, ovariectomized and/or orchiectomized animals,
- in addition cells from cancer cell lines are deposited.

9. A bone system model according to one of claims 1 to 5, **characterised in that** the model is modified as follows:
- the osteoblasts and/or osteoclasts come from normal, ovariectomized and/or orchiectomized animals, osteoporosis therefore being chemically induced *in situ* and/or from transgenic, knock-out animals for any molecules where modulation of the expression induces a reduction in the bone mass.

10. A bone system model according to one of claims 1 to 5, **characterised in that** the model is modified as follows:
- the osteoblasts and/or osteoclasts come from normal animals, osteomalacia therefore being chemically induced *in situ* and/or knock-out for the vitamin D receptor or for any other molecules capable of inducing osteomalacia.

11. A bone system model according to one of claims 1 to 5, **characterised in that** the model is modified as follows:
- the osteoblasts and/or osteoclasts come from normal animals, rheumatoid arthritis therefore being chemically induced *in situ* and/or from transgenic, knock-out animals for any molecules capable of inducing rheumatoid arthritis or from animals having been subjected to type II collagen injections, or any other substances likely to induce articular inflammation mimicking a rheumatoid arthritis.

12. A bone system model according to one of claims 1 to 5, **characterised in that** the model is modified as follows:
- at least one bacterial or viral strain selected among *Enterobacter cloacae,* Staphylococcus aureus, beta-hemolytic streptococcus A, *Haemophilus influenzae type b,* salmonellae, *Pseudomonas,* and/or pneumococci is added into the medium.

13. The use of models according to claims 1 to 5, 8 to 12, for testing the screening of therapeutic molecules.

14. A tumoral cell aggressivity test **characterised in that** tumoral cells of a patient are deposited in a model according to any one of claims 1 to 5, 9 to 12, according to the pathological condition of the patient, in order to observe the development of secondary bone cancer.

15. A toxicity test of a chemical compound **characterised in that** at least one concentration of said compound is tested on a model according to any one of claims 1 to 5, 9 to 12.

## Patentansprüche

1. Modell eines Knochensystems, das eine mineralisierte Matrix und Osteoblaste umfaßt, **dadurch gekennzeichnet, daß** die Osteoblaste auf der Matrix so abgelegt werden, damit ein Konfluenzteppich und/oder Knötchen gebildet werden, und die Osteoklaste werden auf dem besagten Teppich und/oder den besagten Knötchen abgelegt.

2. Modell eines Knochensystems nach Anspruch 1, **dadurch gekennzeichnet, daß** die mineralisierte Matrix eine aus Collagen und Kalziumphosphat und/oder Derivaten von Kalziumphosphat gebildete Matrix ist.

3. Modell eines Knochensystems nach Anspruch 1 oder 2, **dadurch gekennzeichnet, daß** die mineralisierte Matrix eine aus Collagen und Hydroxylapatit gebildete Matrix ist.

4. Modell eines Knochensystems nach einem beliebigen der vorstehenden Ansprüche 1 bis 3, **dadurch gekennzeichnet, daß** das Verhältnis zwischen Osteoklasten / Osteoblasten circa 1/10 bis 1/25 beträgt.

5. Modell eines Knochensystems nach einem beliebigen der vorstehenden Ansprüche 1 bis 3, **dadurch gekennzeichnet, daß** die abgelegten Osteoblaste und/oder Osteoklaste genetisch modifiziert sind.

6. Methode zur Auswahl einer Matrix, mit der ein Modell eines Knochensystems wiederhergestellt werden kann, **dadurch gekennzeichnet, daß** eine Matrix folgendem Verfahren unterzogen wird:
- Ablage eines Teppichs und/oder Osteoblastknötchen mit Konfluenz auf der Matrix;
- Ablage einzelner Osteoklasten auf dem Teppich und/oder den Knötchen;
- Beobachtung der Invasion der Osteoklasten durch den Teppich und/oder die Osteoblastknötchen;
- Auswahl der Matrizes, auf denen die Osteoklaste zwischen der Matrix und dem Teppich und/oder den Osteoblastknötchen lokalisiert sind.

7. Auswahlmethode nach Anspruch 6, **dadurch gekennzeichnet, daß** sie ferner einen Schritt mit Beobachtung der Resorption der Matrix umfaßt, und daß die Matrizes ausgewählt werden, auf denen ebenfalls eine Resorption beobachtet wird.

8. Modell eines Knochensystems nach einem beliebigen der vorstehenden Ansprüche 1 bis 5, **dadurch gekennzeichnet, daß** das Modell wie folgt modifiziert ist:
- Die Osteoblaste und/oder Osteoklaste kommen von normalen, ovariektomierten und/oder orchiektomierten Tieren;
- Es werden außerdem aus Krebszellstämmen hervorgegangene Zellen abgelegt.

9. Modell eines Knochensystems nach einem beliebigen der vorstehenden Ansprüche 1 bis 5, **dadurch gekennzeichnet, daß** das Modell wie folgt modifiziert ist:
- Die Osteoblaste und/oder Osteoklaste kommen von normalen, ovariektomierten und/oder orchiektomierten Tieren, wobei die Osteoporose *in situ* chemisch induziert wird, und/oder Knock-Out Tieren, transgenen für alle Moleküle, deren Expressionsmodulation eine Verringerung der Knochenmasse induziert.

10. Modell eines Knochensystems nach einem beliebigen der vorstehenden Ansprüche 1 bis 5, **dadurch gekennzeichnet, daß** das Modell wie folgt modifiziert ist:
- Die Osteoblaste und/oder Osteoklaste kommen von normalen Tieren, wobei die Osteomalazie *in situ* chemisch induziert wird, und/oder Knock-Out Tieren für den Vitamin D-Rezeptor oder für alle anderen Moleküle, die fähig sind, eine Osteomalazie zu induzieren.

11. Modell eines Knochensystems nach einem beliebigen der vorstehenden Ansprüche 1 bis 5, **dadurch gekennzeichnet, daß** das Modell wie folgt modifiziert ist:
- Die Osteoblaste und/oder Osteoklaste kommen von normalen Tieren, wobei der Gelenkrheumatismus *in situ* chemisch induziert wird, und/oder Knock-Out Tieren, transgenen für alle Moleküle, die fähig sind, einen Gelenkrheumatismus zu induzieren, oder von Tieren, die Collageninjektionen vom Typ II bekommen haben, oder von allen anderen Substanzen, die eine Gelenkentzündung induzieren können, die einen Gelenkrheumatismus nachahmen kann.

12. Modell eines Knochensystems nach einem beliebigen der vorstehenden Ansprüche 1 bis 5, **dadurch gekennzeichnet, daß** das Modell wie folgt modifiziert ist:
- Dem Milieu wird mindestens ein Bakterien- oder Virusstamm hinzugefügt, der unter *Enterobacter cloacae,* dem goldenen Staphylokokkus, dem Betahämolytischen Streptokokkus A, *Haemophilus influenzae Typ b,* den Salmonellen, *Pseudomonas* und/oder den Pneumokokken ausgewählt wird.

13. Benutzung der Modelle nach den Ansprüchen 1 bis 5, 8 bis 12, um die Siebung therapeutischer Moleküle zu testen.

14. Aggressivitätstest von tumorösen Zellen, **dadurch gekennzeichnet, daß** tumoröse Zellen eines Patienten in einem Modell nach einem beliebigen der Ansprüche 1 bis 5, 9 bis 12, je nach pathologischem Zustand des Patienten abgelegt werden, um die Entwicklung von sekundärem Knochenkrebs zu beobachten.

15. Giftigkeitstest einer chemischen Verbindung, **dadurch gekennzeichnet, daß** mindestens eine Konzentration der besagten Verbindung auf einem Modell nach einem beliebigen der vorstehenden Ansprüche 1 bis 5, 9 bis 12 getestet wird.
